# EUROPÄISCHE PATENTANMELDUNG

(11) **EP 1 085 013 A1**
(43) Veröffentlichungstag der Anmeldung: **21.03.2001**
(21) Anmeldenummer: 00118205.4
(22) Anmeldetag: 31.08.2000
(51) Int. Cl.: C07D 209/48, C07D 221/14, C07D 487/04

(54) **Verfahren zur Herstellung cyclischer N-Hydroxy-dicarboximide**

(30) Priorität: 07.09.1999 DE 19942700
(71) Anmelder: Consortium für elektrochemische Industrie GmbH, 81379 München (DE)
(72) Erfinder: Fritz-Langhals, Elke, Dr., 85521 Ottobrunn (DE)
(74) Vertreter: Schuderer, Michael, Dr.

(57) **Zusammenfassung**

Gegenstand der Erfindung ist ein Verfahren zur Herstellung von cyclischen N-Hydroxydicarboximiden, dadurch gekennzeichnet, daß Dicarbonsäuren oder deren Anhydride mit einem Salz des Hydroxylamins in Lösung ohne weiteren Zusatz einer Base umgesetzt werden.

## Beschreibung

Gegenstand der Erfindung ist ein Verfahren zur Herstellung von cyclischen N-Hydroxydicarboximiden.

N-Hydroxydicarboximide werden in der Technik vielfach eingesetzt. Beispielsweise kommen sie bei der Synthese organischer Verbindungen zum Einsatz. Aus EP-A 664479 und JP-A 06301168 ist die Verwendung von N-Hydroxyphthalimid als Oxidationsmittel in photographischen Entwicklern bekannt. Die US-A 5332637 beschreibt die Verwendung von N-Hydroxy-phthalimid in Tonern.

N-Hydroxyphthalimid kommt auch vielfach für die Darstellung von O-substituierten Hydroxylaminen zur Verwendung, die häufig zur Bildung vom Oximen in pharmakologisch und als Pestizide wirksamen Verbindungen eingesetzt werden.

Aus EP-A 25199, EP-A 810318 und US-A 4,709,025 sind Verfahren zur Herstellung antibakteriell hochwirksamer O-substituierter Aminoxy- oder Iminoxyverbindungen, insbesondere aus der Gruppe der Betalactame, mit Hilfe von N-Hydroxyphthalimid bekannt.

WO 95/25090 beschreibt die Verwendung von N-Hydroxydicarboximiden als Starter bei Polymerisationsreaktionen. Aus DE-A 19723961 und DE-A 19723890 ist bekannt, daß, N-Hydroxyphthalimid und dessen Derivate auch für selektive Oxidationsreaktionen eingesetzt werden können.

Für die Synthese von cyclischen N-Hydroxydicarboximiden wurde bislang stets das entsprechende Dicarbonsäureanhydrid und ein Hydroxylammoniumsalz, beispielsweise Hydroxylammoniumchlorid oder Hydroxylammoniumsulfat, unter Zusatz einer Base eingesetzt.

In L.F. Fieser und M. Fieser, "Reagents for Organic Synthesis", Wiley New York, 1967, S. 485-486 und K. Kurita, H. Imajo und Y. Iwakura, J. Polym. Science A-1 **1979**, 17, 1619-1629 wird beispielsweise die Synthese von N-Hydroxyphthalimid unter Einsatz von Pyridin als Base vorgeschlagen.

Aus DE-A 130680, H. Gross und I. Keitel, J. prakt. Chem. **1969**, 311, 692-693 und CN-A 1051170 sind Synthesen von cyclischen N-Hydroxydicarboximiden mittels Hydroxylamin-Hydrochlorid in wäßriger Lösung bekannt, wobei Natriumcarbonat als Base dient.

A. Rougny und M. Daudon, Bull. Soc. Chim. Fr. **1976**, 833-838 beschreiben einen Syntheseweg von N-Hydroxyphthalimid bei dem Natriumacetat als Base eingesetzt wird.

Aus WO 95/25090 ist ein Verfahren zur Herstellung von cyclischen N-Hydroxyimiden unter Verwendung von Natriumhydroxid als Base bekannt.

Von großem Nachteil ist es, daß bei allen beschriebenen Verfahren während der Synthese Salze gebildet werden, die anschließend als Abfallprodukte entsorgt werden müssen. Darüber hinaus ist freies Hydroxylamin schlecht zu handhaben und wird aus diesem Grund nicht eingesetzt. Ein zusätzlicher Nachteil bei den beschriebenen Verfahren ist, daß nur die Dicarbonsäureanhydride, nicht aber die freien Dicarbonsäuren verwendet werden können, da letztere über die Säure-Base-Reaktion rasch deaktiviert werden.

Es bestand daher die Aufgabe ein Verfahren zur Herstellung von cyclischen N-Hydroxydicarboximiden zu finden, welches ohne Zusatz einer Base arbeitet und somit die genannten Nachteile aus dem Stand der Technik nicht besitzt.

Gegenstand der Erfindung ist ein Verfahren zur Herstellung von cyclischen N-Hydroxydicarboximiden, dadurch gekennzeichnet, daß Dicarbonsäuren oder deren Anhydride mit einem Salz des Hydroxylamins in Lösung ohne weiteren Zusatz einer Base umgesetzt werden.

Bei dem erfindungsgemäßen Verfahren werden die entsprechenden Dicarbonsäuren oder deren Anhydride mit einem Salz des Hydroxylamins umgesetzt.

Bei den Salzen des Hydroxylamins handelt es sich um Verbindungen der allgemeinen Zusammensetzung

(H₃NOH)⁺ₘ·H⁺ₙ·X^{(m+n)-}

wobei m die Bedeutung 1, 2 oder 3 besitzt, n die Bedeutung 0, 1 oder 2 besitzt, mit der Maßgabe, daß m+n gleich 1, 2 oder 3 ergeben und X die Bedeutung Phosphat, Sulfat, Nitrat, Halogenid, Acetat, Trifluoracetat, Trichloracetat, Tetrafluorborat oder Sulfonat R^{Y}SO₃⁻ hat.
Der Rest R^{Y} hat die Bedeutung von C₁-C₃-Alkyl, Phenyl oder 4-Methylphenyl.
Die Salze des Hydroxylamins können dabei auch in Form ihrer Hydrate eingesetzt werden.

Bevorzugt werden bei dem erfindungsgemäßen Verfahren Salze des Hydroxylamins aus der Gruppe enthaltend Hydroxylammoniumphosphat, Hydroxylammoniumsulfat, Hydroxylammoniumchlorid oder Hydroxylammoniumtrifluoracetat eingesetzt.

Die Umsetzung der Dicarbonsäuren oder der Dicarbonsäureanhydride wird mit 1 bis 5 Äquivalenten, bevorzugt mit 1 bis 2 Äquivalenten des Hydroxylammoniumsalzes bei Temperaturen zwischen 60 °C und 180 °C, bevorzugt 80 °C bis 150 °C, besonders bevorzugt 100 °C bis 140 °C durchgeführt.

Als Lösungsmittel können gegebenenfalls Wasser oder inerte Solvenzien, beispielsweise aliphatische oder aromatische Kohlenwasserstoffe, Alkohole, chlorierte Kohlenwasserstoffe oder Ether oder auch deren Gemische zugesetzt werden. Die Menge an Lösungsmittel liegt dabei zwischen 10% und 5000% bezogen auf eingesetztes Hydroxylammoniumsalz.

Bevorzugte Lösungsmittel sind Wasser, Methanol, Ethanol, Ligroin, Benzol, Toluol, Nitrobenzol, Xylol oder Methyl-tert-butylether. Die Reaktionszeiten betragen 10 min bis 10 Std., bevorzugt 1 Std. bis 6 Std.

Bei den eingesetzten Dicarbonsäurenanhydriden, oder den durch Zusatz von Wasser daraus erhältlichen Dicarbonsäuren, handelt es sich bevorzugt um Verbindungen, die mindestens einen fünf- oder sechsgliedrigen Ring enthalten. Der Ring kann dabei substituiert oder unsubstituiert vorliegen.

Besonders bevorzugte Dicarbonsäureanhydride sind Verbindungen mit den allgemeinen Formeln Ia, Ib, Ic und Id.

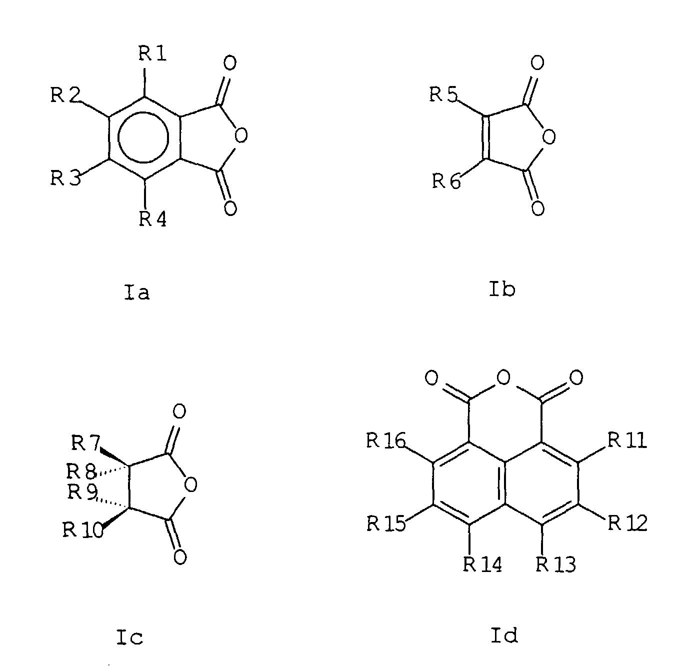

Besonders bevorzugte freie Dicarbonsäuren sind Verbindungen mit den allgemeinen Formeln IIa, IIb, IIc und IId.

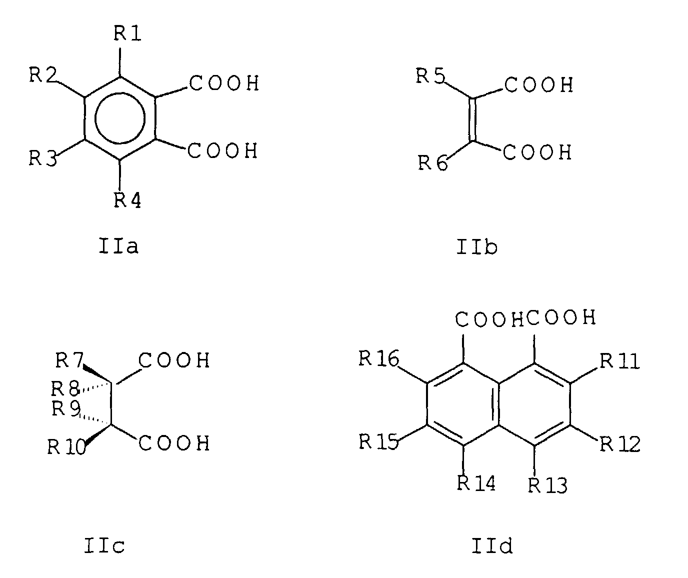

Die Reste R¹ bis R¹⁶ können dabei gleich oder verschieden sein und können Halogenrest, Carboxyrest, Salz oder Ester eines Carboxyrests, Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeuten.

Die Carbamoyl-, Sulfamoyl-, Amino- und Phenylreste können dabei unsubstituiert oder ein- oder mehrfach mit einem weiteren Rest R¹⁷ substituiert sein.

Die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste können gesättigt oder ungesättigt, verzweigt oder unverzweigt vorlegen und mit einem weiteren Rest R¹⁷ ein- oder mehrfach substituiert sein.

Dieser Rest R¹⁷ kann gleich oder verschieden sein und Hydroxy-, Formyl-, Carboxyrest, Ester oder Salz des Carboxyrests, Carbamoyl-, Sulfono- Rest oder Salz des Sulfonorests, Sulfamoyl-, Nitro-, Amino-, Phenyl-, C₁-C₅-Alkyl-, C₁-C₅-Alkoxyrest bedeuten.

Die Reste R⁷ und R⁸ bzw. R⁹ und R¹⁰ dürfen nicht gleichzeitig Hydroxy- oder Aminorest bedeuten.

Jeweils zwei der Substituenten R¹ bis R⁴, R⁵ bis R⁶, R⁷ bis R¹⁰ und/oder R¹¹ bis R¹⁶, können jeweils mit einem Rest -E- verknüpft sein, wobei -E- eine der vier folgenden Bedeutungen hat:

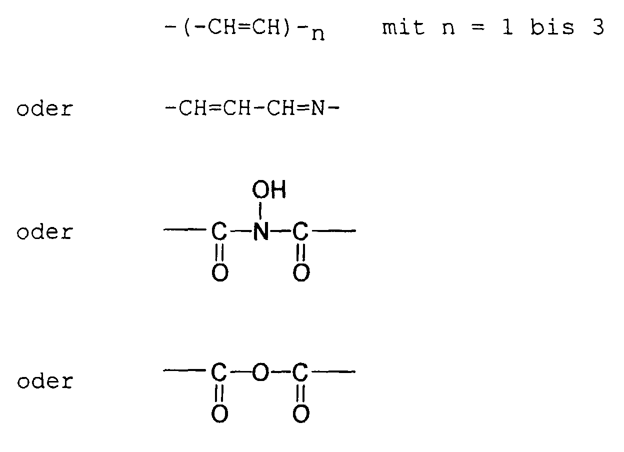

Die Reste R⁷ bis R¹⁰ können auch untereinander durch ein oder zwei Brückenelemente -F- verbunden sein, wobei -F- gleich oder verschieden ist und die Bedeutungen -O-, -S-, -CH₂- oder -CR¹⁸=CR¹⁹- hat. Die Reste R¹⁸ und R¹⁹ können dabei gleich oder verschieden sein und haben die Bedeutung von R¹.

Bei den nach dem erfindungsgemäßen Verfahren erhaltenen cyclischen N-Hydroxydicarboximiden handelt es sich bevorzugt um Verbindungen der allgemeinen Struktur 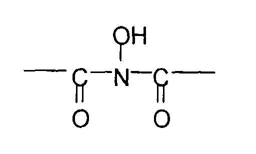 die mindestens einen fünf- oder sechsgliedrigen Ring enthalten. Der Ring kann dabei substituiert oder unsubstituiert vorliegen.

Besonders bevorzugte N-Hydroxydicarboximide sind Verbindungen mit den allgemeinen Formeln IIIa, IIIb, IIIc und IIId.

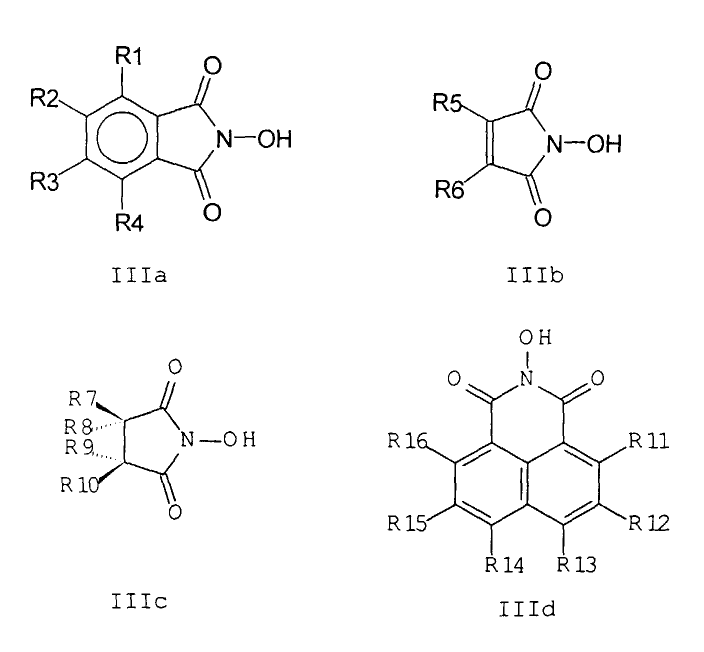

Beispiele für Verbindungen der Formel IIIa sind N-Hydroxyphthalimid sowie substituierte N-Hydroxyphthalimid-Derivate wie 3-Amino-N-hydroxyphthalimid, 4-Amino-N-hydroxyphthalimid, 3-Methyl-N-hydroxyphthalimid, 4-Methyl-N-hydroxyphthalimid, N-Hydroxy-benzol-1,2,4-tricarbonsäureimid, N,N'-Dihydroxy-benzophenon-3,3',4,4'-tetracarbonsäurediimid oder N,N'-Dihydroxypyromellitsäurebisimid.

Beispiele für Verbindungen der Formel IIIb sind N-Hydroxymaleimid sowie substituierte N-Hydroxymaleimid-Derivate wie Pyridin-2,3-dicarbonsäure-N-hydroxyimid, N-Hydroxynaphthalsäureimid sowie ggf. substituierte N-Hydroxynaphthalsäureimid-Derivate.

Beispiele für Verbindungen der Formel IIIc sind N-Hydroxysuccinimid und ggf.substituierte N-Hydroxysuccinimid-Derivate wie z.B. N-Hydroxyweinsäureimid, N-Hydroxy-5-norbornen-2,3-dicarbonsäureimid, exo-N-Hydroxy-7-oxabicyclo[2.2.1]hept-5-en-2,3-dicarboximid, N-Hydroxy-cis-cyclohexan-1,2-dicarboximid und N-Hydroxy-cis-4-cyclohexen-1,2-dicarbonsäureimid.

Beispiele für Verbindungen der Formel IIId sind N-Hydroxynaphthalin-1,8-dicarbonsäureimid, N,N*'*-Dihydroxynaphthalin-1,4,5,8-tetracarbonsäurediimid und N,N*'*-Dihydroxyperylen-3,4,9,10-tetracarbonsäurediimid.

Die folgenden Beispiele dienen zur weiteren Erläuterungen der Erfindung.

### Beispiel 1: Herstellung von N-Hydroxyphthalimid

1.48 g (10.0 mmol) Phthalsäureanhydrid und 0.723 g (3.67 mmol) Hydroxylammoniumphosphat wurden pulverisiert, vermischt und nach Zusatz von 2 ml Wasser ca. 1 1/2 Std. auf 130°C Badtemperatur erhitzt. Die erkaltete zitronengelbe Reaktionsmischung wurde mit Wasser verdünnt, der Feststoff abgesaugt, mit Wasser gewaschen und bei 50°C getrocknet. Die Ausbeute betrug 1.39 g (86 %)

### Beispiel 2: Herstellung von N-Hydroxyphthalimid

0.15 g (1.0 mmol) Phthalsäureanhydrid wurden mit 0.2 bis 1 Äquivalenten Hydroxylammoniumsulfat (s. Tab. 1) vermischt und ggf. nach Zusatz von Wasser (s. Tab.1) in einer geschlossenen Ampulle auf 130°C erhitzt. Die Reaktionszeiten wurden bei den einzelnen Ansätzen entsprechend Tab.1 variiert. Das erkaltete Reaktionsgemisch wurde in d₆-DMSO gelöst und ¹H-NMR-spektroskopisch untersucht. Die Ausbeuten sind jeweils in Tab.1 angegeben.

**Tab.1**

| Umsetzung von Phthalsäureanhydrid mit Hydroxylammoniumsulfat nach Beispiel 2 | | | |
|---|---|---|---|
| Äquivalente | Äquivalente | Rkt.zeit | Ausbeute (%) |
| (H₃NOH)₂SO₄^{a)} | H₂O ^{a)} | (h) | HPI^{b)} |
| 0.2 | 0 | 6 | 44 |
| 0.2 | 0 | 14.5 | 31 |
| 0.2 | 0.1 | 3 | 42 |
| 0.2 | 0.1 | 6 | 50 |
| 0.2 | 0.1 | 14.5 | 45 |
| 0.4 | 0 | 6 | 34 |
| 0.4 | 0 | 14.5 | 41 |
| 0.4 | 0.2 | 3 | 31 |
| 0.4 | 0.2 | 6 | 30 |
| 0.4 | 0.2 | 14.5 | 52 |
| 0.6 | 0 | 6 | 31 |
| 0.6 | 0 | 14.5 | 45 |
| 0.6 | 0.3 | 3 | 25 |
| 0.6 | 0.3 | 6 | 37 |
| 0.6 | 0.3 | 14.5 | 26 |
| 0.8 | 0 | 6 | 43 |
| 0.8 | 0 | 14.5 | 49 |
| 0.8 | 0.4 | 3 | 32 |
| 0.8 | 0.4 | 6 | 31 |
| 0.8 | 0.4 | 14.5 | 32 |
| 1.0 | 0 | 6 | 37 |
| 1.0 | 0 | 14.5 | 34 |
| 1.0 | 0.5 | 3 | 30 |
| 1.0 | 0.5 | 6 | 30 |
| 1.0 | 0.5 | 14.5 | 29 |

| | | | |
|---|---|---|---|
| a) bezogen auf eingesetztes Phthalsäureanhydrid | | | |
| b) bezogen auf eingesetztes N-Hydroxyphthalimid | | | |

### Beispiel 3: Herstellung von 3-Hydroxy-N-hydroxyphthalimid

500 mg (3.05 mmol) 3-Hydroxyphthalsäureanhydrid und 240 mg (1.22 mmol) Hydroxylammoniumphosphat wurden gemischt und mit 1 ml Wasser 3 Std. auf 130°C Badtemperatur erhitzt. Das erkaltete Reaktionsgemisch wurde in Wasser digeriert, abgesaugt und getrocknet. Die Ausbeute betrug 495 mg (91%).
Schmp. 238-239°C (Zers.), ¹H-NMR (CDCl₃ + DMSO): δ = 7.19-7.30 (m, 2H), 7.47-7.59 (mc, 1H), 10.44 (s, breit, OH), ¹H-NMR (CDCl₃): δ = 7.19 (m, 1H), 7.30 (m, 1H), 7.44-7.50 (mc, 1H), 9.24 (s, breit, OH), 10.40 (s, breit, OH), IR (KBr): 3440 m, 3150 w, 1760 w, 1700 s, 1620 m cm⁻¹.

### Beispiel 4: Herstellung von 4-Methyl-N-hydroxyphthalimid

500 mg (3.08 mmol) 4-Methylphthalsäureanhydrid und 243 mg (1.23 mmol) Hydroxylammoniumphosphat wurden gemischt und mit 1 ml Wasser 3 Std. auf 130°C Badtemperatur erhitzt. Die erkaltete Mischung wurde mit 30 ml Wasser digeriert. Der gebildete Niederschlag wurde abgesaugt und bei 60°C im Vakuum getrocknet. Die Ausbeute betrug 437 mg ( 80%).
Schmp. 175-176°C (Zers.), ¹H-NMR (DMSO): δ = 2.48 (s, CH₃), 7.58-7.80 (m, 3H), 10.77 (s, breit, OH).

### Beispiel 5: Herstellung von 4-Nitro-N-hydroxyphthalimid

Analog zu Beispiel 4, jedoch aus 500 mg (2.37 mmol) 4-Nitrophthalsäure (techn., verunreinigt mit ca. 10% 3-Nitrophthalsäure) und 187 mg (0.974 mmol) Hydroxylammoniumphosphat bei einer Badtemperatur von 130°C. Die Ausbeute betrug 332 mg (67%, zu ca. 5% verunreinigt).
Schmelzbereich 101-110°C (Zers.). ¹H-NMR (DMSO): δ = 8.05-8.12 (m, 1H), 8.42-8.49 (m, 1H), 8.59-8.68 (m, 1H).

### Beispiel 6: Herstellung von 3-Nitro-N-hydroxyphthalimid

Analog zu Beispiel 4, jedoch aus 500 mg (2.37 mmol) 3-Nitrophthalsäure und 187 mg (0.947 mmol) Hydroxylammoniumphosphat bei einer Badtemperatur von 130°C. Die Ausbeute betrug 443 mg (90%).
Zersetzung ab 90°C, ¹H-NMR (DMSO): δ = 8.00-8.15 (m, 2H), 8.22-8.31 (m, 1H).

### Beispiel 7: Herstellung von 3-Amino-N-hydroxyphthalimid

1.81 g (10.0 mmol) 3-Aminophthalsäure (Reinheit 90%) und 0.721 g (3.66 mmol) Hydroxylammoniumphosphat wurden pulverisiert, gemischt und mit 2 ml Wasser 3 Std. auf ca. 130°C Badtemperatur erhitzt. Die erkaltete Reaktionsmischung wurde mit wenig Acetonitril aufgeschlämmt und abgesaugt. Die Ausbeute betrug 1.87 g (quantitativ).
Schmp. 247-249°C (Zers.), ¹H-NMR (DMSO): δ = 6.90-7.00 (m, 2H), 7.39-7.46 (m, 1H), IR (KBr): 3470 m, 3380 s, 3180 w, 1770 w, 1710 s, 1640 m, 1585 w cm⁻¹.

### Beispiel 8: Herstellung von N-Hydroxynaphthalin-1,8-dicarbonsäureimid

1.98 g (10.0 mmol) 1,8-Naphthalindicarbonsäureanhydrid und 0.985 g (5.00 mmol) Hydroxylammoniumphosphat wurden mit 2 ml Wasser analog zu Beispiel 7 für 6 Std. bei einer Badtemperatur von 130°C umgesetzt. Das hellbraune Reaktionsgemisch wurde nach dem Erkalten mit Wasser digeriert, der Feststoff abgesaugt und getrocknet. Die Ausbeute betrug 1.89 g (89%). Zersetzung ab 269°C, ¹H-NMR (DMSO): δ = 7.84-7.93 (m, 2H), 8.42-8.53 (m, 4H), 10.76 (s, breit, OH).

### Beispiel 9: Herstellung von Bis-N,N'-dihydroxypyromellitsäureimid

2.18 g (10.0 mmol) Pyromellitsäuredianhydrid und 3.28 g (20.0 mmol) Hydroxylammoniumsulfat wurden gemischt und mit 4 ml Wasser zunächst 3 Std. auf 90°C Badtemperatur und dann weitere 9 Std. auf 110°C Badtemperatur erhitzt. Die ¹H-NMR-spektroskopische Untersuchung ergab einen Umsatz von ca. 36%. ¹H-NMR (DMSO): δ = 8.1 (s, aromat. H)

## Patentansprüche

1. Verfahren zur Herstellung von cyclischen N-Hydroxydicarboximiden, dadurch gekennzeichnet, daß Dicarbonsäuren oder deren Anhydride mit einem Salz des Hydroxylamins in Lösung ohne weiteren Zusatz einer Base umgesetzt werden.

2. Verfahren nach Anspruch 1, dadurch gekennzeichnet, daß es sich bei den Dicarbonsäureanhydriden um Verbindungen mit mindestens einem, ggf. substituierten, fünf- oder sechsgliedrigen Ring, enthaltend die allgemeine Struktur 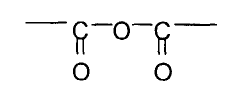 handelt, oder die durch Anlagerung von Wasser hieraus gebildete Dicarbonsäure.

3. Verfahren nach Anspruch 1 bis 2, dadurch gekennzeichnet, daß Dicarbonsäureanhydride mit den allgemeinen Formeln Ia, Ib, Ic und Id oder die denen entsprechenden Säuren eingesetzt werden.

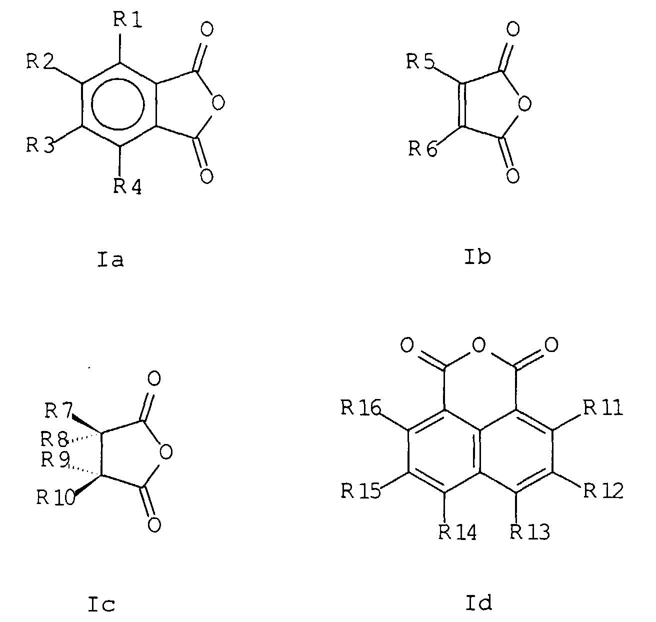

4. Verfahren nach Anspruch 3, dadurch gekennzeichnet, daß die Reste R¹ bis R¹⁶ gleich oder verschieden sind und Halogenrest, Carboxyrest, Salz oder Ester eines Carboxyrests, Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeuten.

5. Verfahren nach Anspruch 4, dadurch gekennzeichnet, daß die Carbamoyl-, Sulfamoyl-, Amino- und Phenylreste unsubstituiert oder ein- oder mehrfach mit einem Rest R¹⁷ substituiert sind, wobei R¹⁷ gleich oder verschieden ist und Hydroxy-, Formyl-, Carboxyrest, Ester oder Salz des Carboxyrests, Carbamoyl-, Sulfono-Rest oder Salz des Sulfono-rests, Sulfamoyl-, Nitro-, Amino-, Phenyl-, C₁-C₅-Alkyl-, C₁-C₅-Alkoxyrest bedeutet, und R⁷ und R⁸ bzw. R⁹ und R¹⁰ nicht gleichzeitig Hydroxy- oder Aminorest bedeutet.

6. Verfahren nach Anspruch 4, dadurch gekennzeichnet, daß die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sind und mit einem Rest R¹⁷ ein- oder mehrfach substituiert sind, wobei R¹⁷ gleich oder verschieden ist und Hydroxy-, Formyl-, Carboxyrest, Ester oder Salz des Carboxyrests, Carbamoyl-, Sulfono-Rest oder Salz des Sulfonorests, Sulfamoyl-, Nitro-, Amino-, Phenyl-, C₁-C₅-Alkyl-, C₁-C₅-Alkoxyrest bedeutet, und R⁷ und R⁸ bzw. R⁹ und R¹⁰ nicht gleichzeitig Hydroxy- oder Aminorest bedeutet.

7. Verfahren nach Anspruch 3 bis 6, dadurch gekennzeichnet, daß jeweils zwei der Substituenten R¹ bis R⁴, R⁵ bis R⁶, R⁷ bis R¹⁰, und/oder R¹¹ bis R¹⁶ zu einem Rest -E- verknüpft sind, wobei -E- die Bedeutung 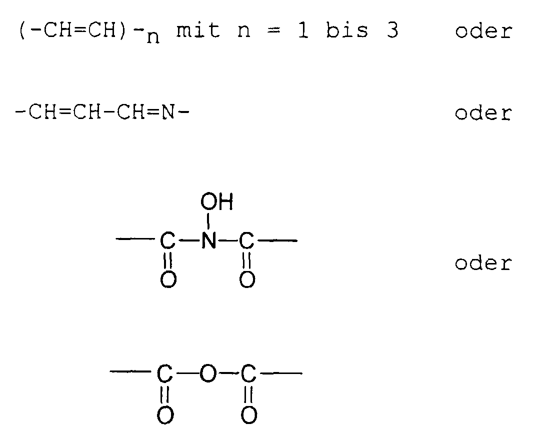 hat.

8. Verfahren nach Anspruch 3 bis 7, dadurch gekennzeichnet, daß die Reste R⁷ bis R¹⁰ untereinander durch ein oder zwei Brückenelemente -F- verbunden sind, wobei -F- gleich oder verschieden ist und die Bedeutungen -O-, -S-, -CH₂- oder -CR¹⁸=CR¹⁹- hat.

9. Verfahren nach Anspruch 8, dadurch gekennzeichnet, daß R¹⁸ und R¹⁹ gleich oder verschieden sind und die Bedeutung von R¹ haben.

10. Verfahren nach Anspruch 1 bis 9, dadurch gekennzeichnet, daß die Salze des Hydroxylamins die allgemeine Zusammensetzung
(H₃NOH)⁺ₘ·H⁺ₙ·X^{(m+n)-}
besitzen, wobei m die Bedeutung 1, 2 oder 3 hat, n die Bedeutung 0, 1 oder 2 hat, mit der Maßgabe, daß m+n gleich 1, 2 oder 3 ergibt, und X einen oder mehrere der Gruppen umfassend Phosphat, Sulfat, Nitrat, Halogenid, Acetat, Trifluoracetat, Trichloracetat, Tetrafluorborat oder Sulfonat R^{Y}SO₃⁻ bedeutet und der Rest R^{Y} dabei die Bedeutung C₁-C₃-Alkyl, Phenyl oder 4-Methylphenyl besitzt.

11. Verfahren nach Anspruch 1 bis 10, dadurch gekennzeichnet, daß die Salze des Hydroxylamins in Form ihrer Hydrate eingesetzt werden.

12. Verfahren nach Anspruch 1 bis 11, dadurch gekennzeichnet, daß ein oder mehrere Salze des Hydroxylamins aus der Gruppe enthaltend Hydroxylammoniumphosphat, Hydroxylammoniumsulfat, Hydroxylammoniumchlorid oder Hydroxylammoniumtrifluoracetat eingesetzt werden.

13. Verfahren nach Anspruch 1 bis 12, dadurch gekennzeichnet, daß die Umsetzung bei einer Temperatur zwischen 60 °C und 180 °C erfolgt.

14. Verfahren nach Anspruch 1 bis 13, dadurch gekennzeichnet, daß als Lösungsmittel ein oder mehrere Substanzen aus der Gruppe umfassend Wasser, aliphatische Kohlenwasserstoffe, aromatische Kohlenwasserstoffe, Alkohole, chlorierte Kohlenwasserstoffe oder Ether verwendet werden.

15. Verfahren nach Anspruch 1 bis 14, dadurch gekennzeichnet, daß als Lösungsmittel ein oder mehrere Substanzen aus der Gruppe enthaltend Wasser, Methanol, Ethanol, Ligroin, Benzol, Toluol, Nitrobenzol, Xylol oder Methyl-tert-butylether verwendet werden.
